# EUROPEAN PATENT APPLICATION

(11) **EP 1 688 103 A1**
(43) Date of publication of application: **09.08.2006**
(21) Application number: 06101246.4
(22) Date of filing: 03.02.2006
(51) Int. Cl.: A61C 8/00, A61B 17/86

(54) **Screws for attaching bone grafts in jaw implants**

(30) Priority: 04.02.2005 IT BO20050054
(71) Applicant: Cizeta Surgical S.r.l., 40068 San Lazzaro di Savena (IT)
(72) Inventor: PINI, Vittorio, 40065, PIANORO (IT)
(74) Representative: Jorio, Paolo

(57) **Abstract**

A screw (100, 101) for attaching bone grafts (T) in jaw implants. The screw (100, 101) comprises a shank (102) and a head (103). The screw (100, 101) is characterised in that said head (103) presents an under-head surface (104) substantially perpendicular to the longitudinal symmetrical axis (a) of the screw (100, 101). Also described is a jaw implant comprised of a bone graft (T) and at least one screw (100, 101) of an innovative type.

## Description

The present invention relates to a screw for fixing bone grafts in jaw implants. Alveolar regeneration is a well-consolidated surgical practice used to substitute the lack of bone in the part of the jaw where a dental implant will be inserted at a later time. The alveolar generation operation is part of so-called pre-prosthesis surgery.

Normally for alveolar regeneration, a bone graft is taken from the iliac ridge or the fibula of the same patient (homograft), the graft is shaped appropriately and positioned on the jaw (the so-called on-lay graft).

The graft is then attached to the jaw using a metal plate screwed in with one or more screws. These screws are then removed after approximately 3-5 months after the positioning of the graft, once it has become totally solid with the rest of the jaw as a result of the forming of bone tissue, which, in other words, "encloses" it in the jaw.

In addition, it should be noted that often the bone graft is taken from the jaw itself in the jaw ramus area.
Furthermore, traditional osteosynthesis screws with 1.0-1.6 mm diameters and lengths that vary from 10 mm to 20 mm are commonly used in order to attach the bone graft and the metal plate.

However, screws of prior art present under-head camber in order to adhere perfectly in the flared hole on the relative metal plate. Furthermore these screws also have a low profile (flat) head on the upper side to ensure less invasion.
However, unfortunately under-head flaring on traditional screws of this type creates a compression point directly on the bone graft.

More particularly, since naturally the metal plate includes a hole for the insertion of the fixing screw, the flared form of the screw is inserted into the hole in the metal plate and into the hole of the bone implant (obviously both bone implant and metal plate holes being coaxial) that can lead to possible necrosis and simultaneous reabsorb ion of the screw in the bone itself.

Furthermore, traditional fixing screws of the type describe above pose a further problem in that they are prone to breaking inside the bone implant during screwing action, and often newly formed bone can cover the head of the screw, because of its deliberately flat form.

Therefore the aim of the present invention is to create a screw for attaching bone grafts in jaw implants without the disadvantages described above.

For this purpose, a screw for attaching bone grafts in jaw implants is realised according to the present invention, and according to the characteristics described in claim 1 and in the relative dependent claims.

The present invention will now be described with reference to the appended drawings, which illustrate non-limiting embodiments wherein:
- figure 1 shows a jaw in which a bone graft has been fixed by means of a pair of screws, object of the present invention;
- figure 2 shows an isometric view of the screw, object of the invention;
- figure 3 shows a side view of the screw in figure 2;
- figure 4 illustrates a screw according to the invention wherein the screw head diameter is 5 mm; and
- figure 5 illustrates a screw according to the invention wherein the screw head diameter is 3 mm.

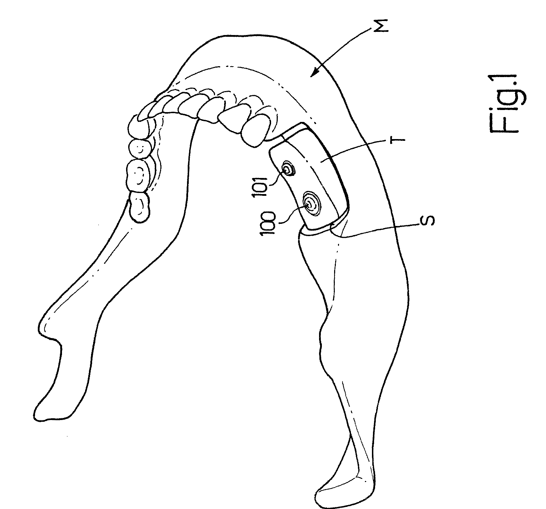

In figure 1 the letter M refers to the jaw of a patient.

A hollow cavity "S" has been surgically performed on the jaw "M", inside which the bone graft "T" will be inserted by means of two screws 100, 101 having different diameters and lengths, both realised according to the principles of the present invention.

Using suitable devices, the surgeon drills coaxial holes for each screw 100, 101 (not shown) in the jaw M and in the graft T. One of the two screws 100, 101 is screwed into each pair of coaxial holes (not shown).

In the situation shown in figure 1, the bone graft T has been inserted and screwed to the rest of the jaw M to permit bone tissue formation (in 3-5 months) that will weld the jaw M and the graft T together. The bone tissue that will form in the following 3-5 months will provide a type of natural "gluing action" of graft T to jaw M.

Once again, as shown in figure 1, because of the particular configuration of the screws 100, 101 (described further on) no metal plate is required between the heads of screws 100, 101, and the upper surface of graft T.

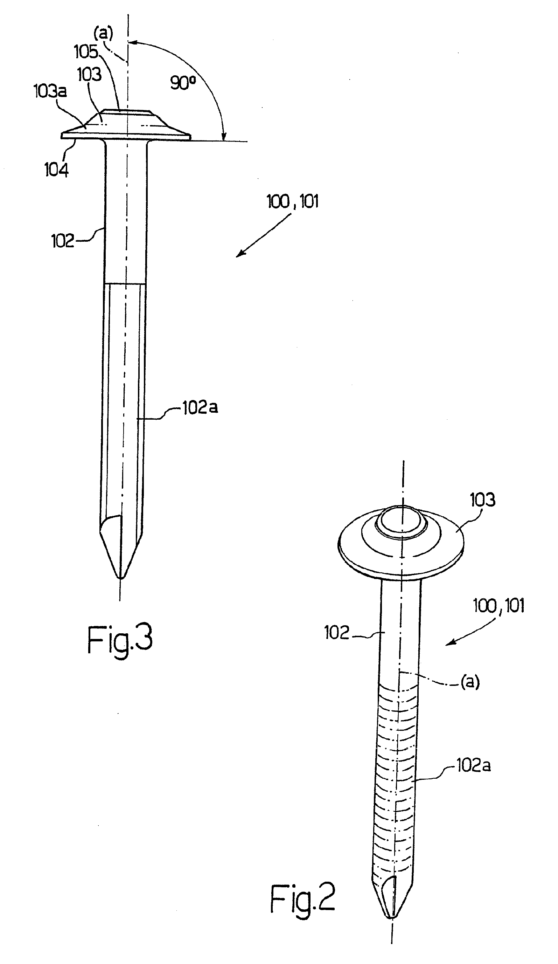

A complete screw 100, 101 is illustrated in figures 2 and 3.

For example, the screw 100 (but this applies to screw 101 in exactly the same manner) is composed of a shank 102 topped by a head 103.

Both the shank 102 and the head 103 have a longitudinal symmetrical axis (a).

According to the present invention the head 103 of screw 100 has a basically "mushroom" or "hat" form that in turn presents an under-head surface 104 which is substantially perpendicular to axis (a).

In other words the support surface of the head 103 in contact with the upper surface of the bone graft T is an under-head surface 104, substantially at a 90° angle with the axis (a) being in longitudinal symmetry with screw 100.

In this manner, the support surface of the head 103 of screw 100 on graft T is quite large in order to prevent the aforesaid enclosing action of a portion of head 103 by the bone tissue of graft T.

Because of the width of the support surface of head 103 it is possible to avoid the use of a metal plate, as shown in figure 1.

As was stated previously, after a few months, the screws 1000, 101 are removed, while the bone graft T remains inside the cavity S welded in a perfectly natural manner to the rest of the jaw M. Naturally, immediately after the removal of the screws 100, 101, the dental implant can be mounted on the graft T (not shown).

Reconsidering screw 100 in figures 2 and 3 it is possible to see that a portion 102a is threaded. In an advantageous way, portion 102a comprises a double threading which, as is well known, permits the screw 100 to be inserted with only half the number of turns by a screwdriver (not shown).

Again, in figures 2 and 3, the head 103 presents a portion 103a in "mushroom" or "hat" form that projects outwards towards the exterior, in other words, from the opposite part of the shank 102 in relation to the under-head surface 104.

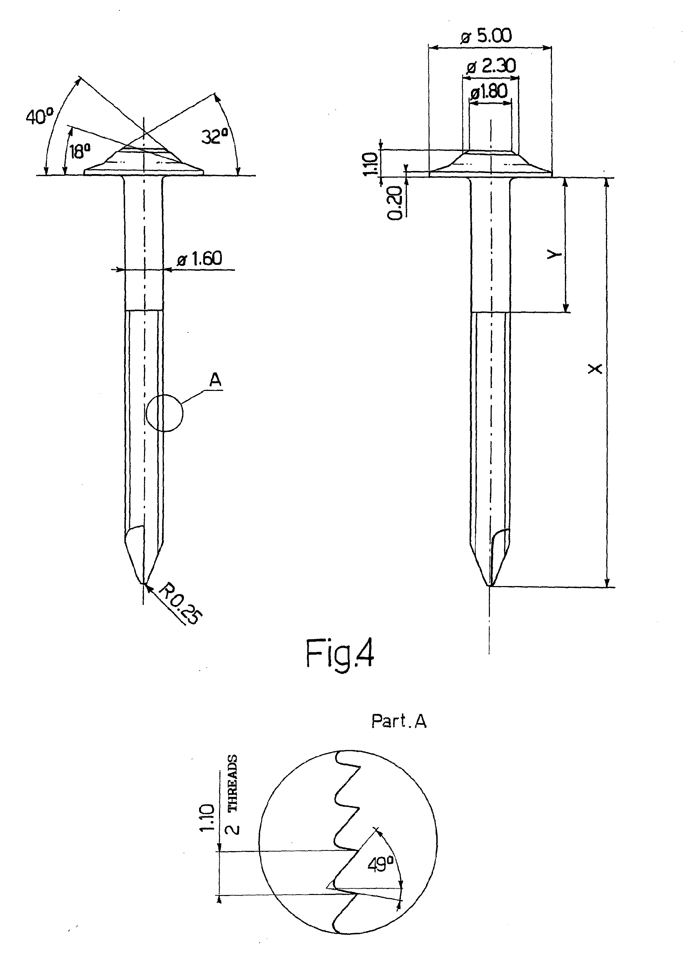

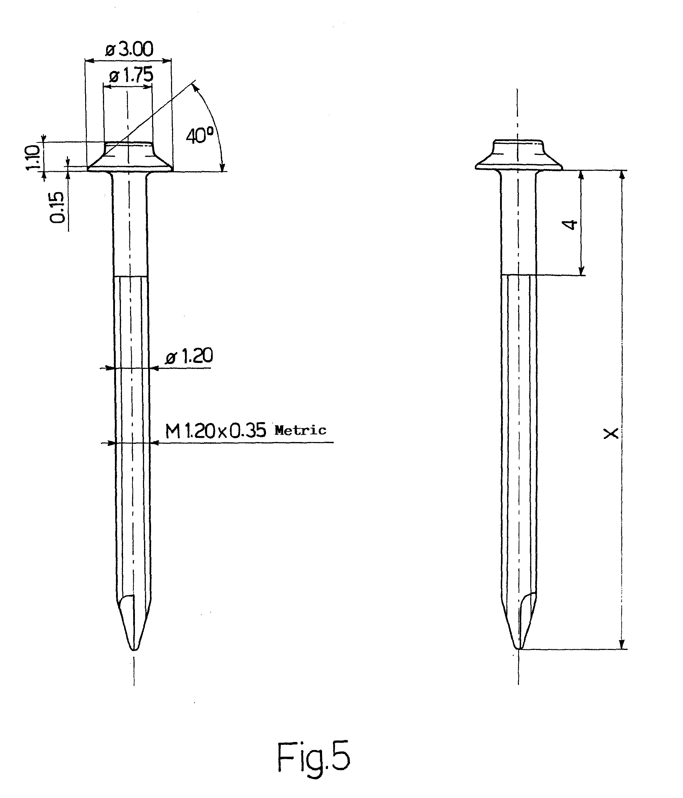

Advantageously the portion 103a tapers according to certain advantageous parameters that will be described further on in the present description (see figures 4, 5 further on).

Moreover, portion 103a comprises a terminal surface 105, parallel to the under-head surface 104, on which a seat (not shown) has been drilled for the insertion of the prismatic tip of a screwdriver (not shown) during the screwing and unscrewing of screw 100.

Advantageously, but not necessarily, said screws are available with two different shank diameters (1.6 mm and 1.2 mm) which correspond respectively with a diameter at the base of the head, 5.0 mm (figure 4, further on) and 3.0 mm (figure 5, further on).

Moreover, according to diameter, the length of the screw can also vary between 8mm and 18 mm (see further on, figures 4 and 5) and these require dedicated drills and screwdrivers.

In order to obtain the best possible attaching of bone graft T on jaw M, screws must respect, advantageously, the following ratio between the main measurements:

In the case of the screw shown in figure 4 with a ∅ of 5 mm at the base of the head, advantageously, but not necessarily, the total length X of the screw shank (from the under-head surface to the tip of the screw) is 10 - 12 - 14 - 16 - 18 mm.

Again in the case of figure 4, the head preferably presents a thickness of 1.10 mm, flat on the upper surface, with a centre seat having a ∅ of 1.80 mm; the upper flat surface is attached to the under-head surface with triple angle of 18°, 32° and 40°.

The shank diameter (web plus threading) is advantageously 1.6 mm.

The length X of the screw shank, partially threaded, must advantageously respect the following ratios:
- When the length X of the shank is 10 - 12 - 14 mm: Y=3.5 mm (non-threaded part under the head);
- When the length X of the shank is 16 mm: Y=5.0 mm (non-threaded part under the head); and
- When the length X of the shank is 18 mm: Y=6.0 mm (non-threaded part under the head).

In the case of the screw shown in figure 5 with a Ø of 3 mm at the base of the head, advantageously the total length of the screw shank (from the under-head surface to the tip of the screw) is 8 - 10 - 12 - 14 - 16 mm.

In this case, the head advantageously presents a thickness of 1.10 mm, flat on the upper surface, with a centre seat having a ∅ f 1.75 mm, attached to the under-head surface with an angle of 40°.

The shank diameter (web plus threading) is advantageously 1.2 mm.

The length X of the screw shank, partially threaded, must advantageously respect the following ratios:
- When the length X of the shank is 8 - 10 - 12 - 14 - 16 mm: Y=4.0 mm (non-threaded part under the head).

As stated previously the screw must advantageously be double threaded. The most advantageous step is 1.10 mm with a thread angle of 49°.

The screw, object of the present invention can be advantageously made from titanium.

The advantages of the present screw for attaching bone grafts in jaw implants are as follows:
- not having a flared shape, but the under-head surface being completely flat, there is no danger that a portion of the screw will insert itself in the bone graft hole; in this manner all risk of necrosis can be prevented;
- since the head of the screw is protruding, this prevents the creation of bone formation on the head of the screw;
- the screw configuration is very strong and it is almost impossible to break;
- the screw is very easy to screw in because of the double threading; and
- the absence of the metal plate makes the implant much lower in cost.

## Claims

1. Screw (100, 101) for attaching bone grafts (T) in jaw implants; screw (100, 101) comprising a shank (102) and a head (103); screw (100, 101) **characterised in that** said head (103) presents an under-head surface (104) substantially perpendicular to the longitudinal symmetrical axis (a) of the screw (100, 101).

2. Screw (100, 101) according to claim 1, **characterised in that** said head (103) presents a portion (103a) that protrudes from the opposite part of said shank (102) in relation to said under-head surface (104).

3. Screw (100, 101) according to any one of the previous claims, **characterised in that** it is double threaded.

4. Screw (100, 101) according to claim 3, **characterised in that** it presents a step of 1.10 mm with a thread angle of 49°.

5. Screw (100, 101) according to any one of the previous claims, wherein the head has a diameter of 5 mm, the following ratios are foreseen:
- when the length X of the shank is 10 - 12 - 14 mm: Y=3.5 mm (non-threaded part under the head);
- when the length X of the shank is 16 mm: Y=5.0 mm (non-threaded part under the head); and
- when the length X of the shank is 18 mm: Y=6.0 mm (non-threaded part under the head).

6. Screw (100, 101) according to claim 5, wherein the head has a thickness of 1.10, the upper flat surface being attached to the surface under the head with a triple angle of 18°, 32° e 40°.

7. Screw (100, 101) according to any one of the claims 1-4, wherein a head with a diameter of 3 mm will have the following ratio:
- when the length X of the shank is 8 - 10 - 12 - 14 - 16 mm: Y=4,0 mm (non-threaded part under the head).

8. Screw (11, 101) according to claim 7, wherein the head has a thickness of 1.10, the upper flat surface being attached to the surface under the head with a 40°.

9. Use in a jaw implant of a screw (100, 101) according to any one of the previous claims.

10. Jaw implant comprising a bone graft (T) and at least one screw (100, 101) according to any one of the claims 1-8.
